# EUROPÄISCHE PATENTSCHRIFT

(11) **EP 0 763 030 B1**
(45) Veröffentlichungstag und Bekanntmachung des Hinweises auf die Patenterteilung: **07.01.1999**
(21) Anmeldenummer: 95921795.1
(22) Anmeldetag: 31.05.1995
(51) Int. Cl.: C07D 309/04, C07D 307/12

(54) **VERFAHREN ZUR HERSTELLUNG VON HETEROCYCLISCHEN ALDEHYDEN**
PROCESS FOR PREPARING HETEROCYCLIC ALDEHYDES
PROCEDE DE PREPARATION D'ALDEHYDES HETEROCYCLIQUES

(30) Priorität: 03.06.1994 DE 4419514
(43) Veröffentlichungstag der Anmeldung: 19.03.1997
(73) Patentinhaber: BASF AKTIENGESELLSCHAFT, 67056 Ludwigshafen (DE)
(72) Erfinder: SCHNURR, Werner, D-67273 Herxheim (DE); FISCHER, Rolf, D-69121 Heidelberg (DE); WULFF-DÖRING, Joachim, D-67227 Frankenthal (DE); IRGANG, Matthias, D-69121 Heidelberg (DE); NEUHAUSER, Horst, D-67373 Dudenhofen (DE)
(86) Internationale Anmeldenummer: EP9502071
(87) Internationale Veröffentlichungsnummer: WO9533741

(56) Entgegenhaltungen:
- EP-A- 0 343 640

## Beschreibung

Die vorliegende Erfindung betrifft ein Verfahren zur Herstellung von heterocyclischen Aldehyden der allgemeinen Formel I 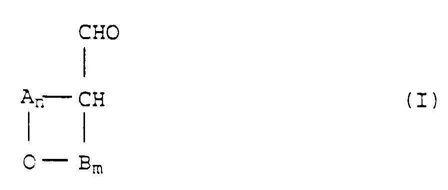 in der die Variablen A und B für Methylengruppen, die ein oder zwei unter den Reaktionsbedingungen inerte Substituenten tragen können, und m und n für 1 bis 5 stehen, wobei die Summe aus m und n größer als 2 ist.

Aldehyden der allgemeinen Formel I, in welchen die Formylgruppe an einen durch ein Sauerstoffatom unterbrochenen gesättigten Ring gebunden ist, kommt als Zwischenprodukten große Bedeutung zu. So ist 4-Formyltetrahydropyran beispielsweise ein Baustein für Herbizide vom Cyclohexenontyp (vgl. z.B. EP-A 142 741).

Die Herstellung von Aldehyden aus Carbonsäuren oder Carbonsäureestern wird in der Technik üblicherweise in zwei Stufen durchgeführt, indem man die Ausgangsstoffe zunächst bis zur Alkoholstufe reduziert und dann eine Oxidation zum Aldehyd vornimmt. Gut untersucht ist etwa die Herstellung von 4-Formyltetrahydropyran aus Tetrahydropyran-4-carbonsäuremethylester (vgl. EP-A 546 396 und DE-A 40 39 918).

Aus der EP-A 439 115 ist bekannt, aliphatische oder alicyclische Carbonsäuren, z.B. Cyclohexancarbonsäure, in Gegenwart eines Chrom-Zirkoniumdioxid-Katalysators zu den entsprechenden Aldehyden zu hydrieren.

In der EP-A 343 640 wird die Hydrierung von heteroaromatischen Carbonsäuren zu den entsprechenden Aldehyden beschrieben, wobei der Katalysator hierfür mindestens ein Zink-, Yttriumoxid, ein Oxid der Gruppe der Lanthaniden oder ein Oxid eines Elements der Gruppe 4A des Periodensystems wie Zirkoniumdioxid enthalten soll. Bezüglich gesättigter Heterocyclen wird keine Aussage gemacht.

Der Erfindung lag die Aufgabe zugrunde, die Carbonsäuren IIa bzw. die Ester IIb unmittelbar partiell zu den heterocyclischen Aldehyden I zu hydrieren, d.h. ohne den bisher beschrittenen Umweg über die Alkohole und die sich daran anschließende Oxidation zu den Aldehyden.

Demgemäß wurde ein Verfahren zur Herstellung der heterocyclischen Aldehyde I gefunden, welches dadurch gekennzeichnet ist, daß man eine Carbonsäure der allgemeinen Formel IIa 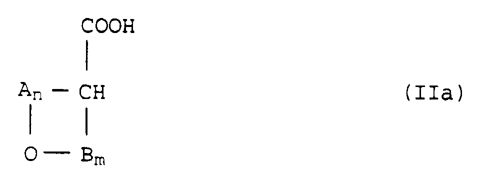 oder einen ihrer Ester IIb, welcher sich von einem C₁- bis C₁₀-Alkohol ableitet, bei 200 bis 450°C an einem Katalysator hydriert.

Die für die Umsetzung nach dem erfindungsgemäßen Verfahren geeigneten Carbonsäuren IIa und Ester IIb können am Ring vor allem einen bis drei unter den Reaktionsbedingungen inerte Substituenten (R) tragen, insbesondere C₁- bis C₃-Alkyl- oder -Alkoxygruppen.

Zu den bevorzugten Verfahrensprodukten I gehören diejenigen, welche sich von Carbonsäuren IIa oder deren Estern IIb ableiten, in denen der das Sauerstoffatom enthaltende Ring 5 bis 7 Ringglieder aufweist, d.h. bei denen die Summe aus n und m 3 bis 5 beträgt, wobei einer oder zwei der Reste R für eine C₁- bis C₃-Alkylgruppe und die übrigen Reste R für Wasserstoff stehen. Derartige Verbindungen I sind vor allem 3-Formyl-2-methyltetrahydrofuran, 3-Formyl-2,4-dimethyltetrahydrofuran, 2-Ethyl-4-formyltetrahydrofuran, 3-Formyl-5-iso-propyltetrahydropyran und 3-Formyl-2,7-Dimethyloxepan. Besonders bevorzugt sind solche derartigen Verfahrensprodukte I, in denen der Heterocyclus unsubstituiert ist, wie in 3-Formyltetrahydrofuran und vor allem in 4-Formyltetrahydropyran.

Vorzugsweise verwendet man aus der Gruppe der als Ausgangsstoffe geeigneten Ester IIb solche, die sich von C₁- bis C₁₀-Monoalkoholen ableiten. Hierunter sind vor allem solche Ester IIb geeignet, in denen die Monoalkoholkomponente einen unsubstituierten C₁- bis C₁₀-Kohlenwasserstoffrest aufweist, wie im Falle
- der ganz besonders bevorzugten C₁- bis C₁₀-Alkanole, insbesondere der C₁- bis C₈-Alkanole wie vor allem Methanol, Ethanol, n-Propanol, 1-Methylethanol, n-Butanol, 1-Methylpropanol, 2-Methylpropanol und 1,1-Dimethylethanol und daneben n-Pentanol, n-Hexanol, n-Heptanol, n-Octanol und 2-Ethylhexanol und daneben
- der C₃- bis C₁₀-Cycloalkanole, vorzugsweise der C₃- bis C₈-Cycloalkanole wie vor allem Cyclopentanol und Cyclohexanol und daneben 2-Methylcyclohexanol, 2,6-Dimethylcyclohexanol, Cycloheptanol und Cyclooctanol,
- der C₆- bis C₁₀-aromatischen Monoalkohole, vorzugsweise Phenol und daneben der Naphthole oder
- der C₇- bis C₁₀-Aralkanole, vorzugsweise der Phenyl-(C₁-C₃)-alkanole wie vor allem Phenylmethanol, 2-Phenylethanol und 3-Phenylpropan-1-ol und daneben 1-Phenylethanol, 2-Phenylpropan-1-ol und 1-Phenylpropan-1-ol.

Im Falle der Herstellung von 4-Formyltetrahydropyran geht man insbesondere von Tetrahydropyran-4-carbonsäure, ihrem Methyl- oder Ethylester aus.

Die Carbonsäuren IIa bzw. die Ester IIb sind zum Teil käuflich, oder sie sind in an sich bekannter Weise erhältlich (vgl. Houben-Weyl, Methoden der Organischen Chemie, 4. Auflage, Thieme Verlag, Stuttgart, Band 8, Seite 365 ff. bzw. Seite 508 ff.).

Die erfindungsgemäß geeigneten Katalysatoren bestehen vorzugsweise, und zwar insbesondere zu 75 bis 100 und vor allem zu 90 bis 100 Gew.-%, aus Zirkoniumdioxid und/oder einem oder mehreren Manganoxiden. Das verwendete Zirkoniumdioxid hat in der Regel eine BET-Oberfläche von 5 bis 150, vorzugsweise 20 bis 130 und insbesondere 40 bis 120 m²/g, wobei unter der BET-Oberfläche die nach der Brunauer-Emmett-Teller-Methode bestimmte Katalysator-Oberfläche zu verstehen ist (vgl. Z. Anal. Chem. 238, Seite 187 (1968)). Vorzugsweise verwendet man monoklines Zirkoniumdioxid und daneben solches mit kubischem oder tetragonalem Kristallgitter.

Unter den Manganoxiden kommen vor allem Mangan-(II)-oxid und daneben Mangan-(III)-oxid und Mangan-(IV)-oxid sowie deren Gemische in Betracht. Die BET-Oberflächen liegen hier in der Regel bei 5 bis 60, vorzugsweise 10 bis 50 m²/g.

In einer bevorzugten Ausführungsform des erfindungsgemäßen Verfahrens verwendet man einen Katalysator, welcher neben Zirkoniumdioxid und/oder einem oder mehreren Manganoxiden 0,1 bis 10 und vor allem 0,5 bis 5 Gew.-% einer oder mehrerer Verbindungen des Chroms, Yttriums und/oder eines oder mehrerer Elemente der Lanthaniden-Reihe des Periodensystems der Elemente (im folgenden "Lanthanid-Elemente") enthält, wobei die Verbindungen der Lanthanid-Elemente bevorzugt sind.

Zu den Lanthanid-Elementen zählen Lanthan, Cer, Praseodym, Neodym, Promethium, Samarium, Europium, Gadolinium, Terbium, Dysprosium, Holmium, Erbium, Thulium, Ytterbium und Lutetium.

Vor allem die Verbindungen des Lanthans und Praseodyms, und daneben die des Cers, Samariums, Neodyms und Europiums, eignen sich als Bestandteile der erfindungsgemäß verwendeten Katalysatoren.

Man verwendet vorzugsweise solche Katalysatoren, in denen das Chrom bzw. aie Lanthanid-Elemente zumindest teilweise, und vor allem zu mehr als 80 Gew.-% in oxidischer Form vorliegen.

Bei der Herstellung der Katalysatoren werden daher diese Elemente vorzugsweise als Oxide eingesetzt oder in Form derjenigen ihrer Verbindungen, welche durch Erhitzen leicht, gegebenenfalls in Gegenwart von Wasser und/oder Sauerstoff, in die Oxide überführt werden können.

Hierzu gehören im Falle der Lanthanid-Elemente vor allem die dreiwertigen Oxide wie Cer-(III)-oxid und Lanthan-(III)-oxid und daneben die Chloride und Nitrate wie Lanthan-(III)-nitrat, Cer-(III)-nitrat, Praseodym-(III)-chlorid und Samarium-(III)-chlorid sowie Verbindungen mit organischen Anionen wie die Acetate und die Oxalate, z.B. Cer-(III)-oxalat.

Im Falle des Chroms kommen vor allem Chrom-(III)-oxid sowie die Chloride, Nitrate und Acetate des dreiwertigen Chroms, insbesondere Chrom-(III)-chlorid und Chrom-(III)-nitrat, für die Herstellung der erfindungsgemäß geeigneten Katalysatoren in Betracht.

In einer besonders bevorzugten Ausführungsform des erfindungsgemäßen Verfahrens verwendet man einen Katalysator, welcher im wesentlichen aus Zirkoniumdioxid besteht und 0,1 bis 10, insbesondere 0,5 bis 5 Gew.-% einer Verbindung eines oder mehrerer Lanthanid-Elemente, vor allem Lanthan-(III)-oxid, enthält.

Im übrigen haben Spuren vor allem der Elemente Eisen, Aluminium, Silicium und Magnesium mit einem Gewichtanteil von in der Summe weniger als 2 Gew.-%, bezogen auf das Gesamtgewicht des Katalysators, keinen erkennbar abträglichen Einfluß auf das gute Gelingen des erfindungsgemäßen Verfahrens.

Diese Katalysatoren, sofern nicht käuflich, sind in an sich bekannter Weise erhältlich (vgl. z.B. Catal. Rev. - Sci. Eng. 27, Seite 341 bis Seite 372 (1985)).

Die Katalysatoren können als Trägerkatalysatoren auf einem an sich bekannten inerten Träger wie Siliciumdioxid oder Aluminiumoxid und vor allem als Vollkatalysatoren eingesetzt werden. Sie eignen sich in Form von Strängen, vorzugsweise von 1 bis 5 mm längstem Durchmesser, Pellets oder Kugeln.

Die Mengenangaben über die Zusammensetzung der Katalysatoren beziehen sich auf deren aktive Masse, aber ohne Berücksichtigung der Trägermaterialien.

Die Hydrierung der Carbonsäuren IIa oder Ester IIb führt man vorzugsweise bei Temperaturen von 250 bis 400, in der Regel von 300 bis 380°C und Drücken von 0,1 bis 20, vorzugsweise von 0,8 bis 5 bar und insbesondere bei Normaldruck durch.

Im allgemeinen arbeitet man ferner bei einer Katalysatorbelastung von 0,01 bis 10, vorzugsweise 0,01 bis 3 kg der Ausgangsverbindung II pro Kilogramm Katalysator und Stunde.

Vorzugsweise richtet man es so ein, daß während der Hydrierung pro Mol II 2 bis 100 mol, besonders 10 bis 70 mol Wasserstoff zugegen sind.

Aus Sicherheitsgründen kann dem Wasserstoff ein Inertgas, vor allem Stickstoff oder Wasserdampf und daneben Argon zugemischt werden.

Besonders die festen Verbindungen II können in flüssiger Phase in einem unter den Hydrierbedingungen inerten Lösungsmittel wie Kohlenwasserstoffen, z.B. n-Hexan oder Cyclohexan, oder Wasser, vor allem aber lösungsmittelfrei umgesetzt werden.

Bei Umsetzungen in der Gasphase werden die Verbindungen II zweckmäßigerweise dampfförmig und gegebenenfalls mittels eines Trägergasstroms in die Reaktionszone gebracht. Bevorzugtes Trägergas ist Stickstoff.

Vorzugsweise nimmt man die Hydrierung der Verbindungen II kontinuierlich und vor allem in der Gasphase nach den hierfür bekannten Techniken, insbesondere an einem Katalysator-Festbett, vor.

In einer weiteren bevorzugten Ausführungsform, die auch für sich allein besonders vorteilhaft ist, geht man zur Herstellung von 4-Formyltetrahydropyran von Tetrahydropyran-4-carbonsäure oder einen ihrer Ester, welcher sich von einem C₁- bis C₁₀-Alkohol ableitet, aus und führt die Hydrierung in Gegenwart eines Katalysators durch, welcher im wesentlichen aus Zirkoniumdioxid besteht und 0,1 bis 5 Gew.-% Lanthan-(III)-oxid enthält, wobei man vorteilhafterweise bei 300 bis 380°C und einem Druck von 0,8 bis 5 bar arbeitet.

Die Aufarbeitung der rohen Reaktionsgemische auf das Verfahrensprodukt I kann in an sich bekannter Weise erfolgen, vor allem gegebenenfalls durch destillatives Entfernen des Lösungsmittels und Reinigen des Rohprodukts, etwa mittels Destillation oder Umkristallisation.

Die Katalysatoren können in den meisten Fällen durch Erhitzen auf 300 bis 450°C im Sauerstoff- oder Luftstrom regeneriert werden.

Bei der Hydrierung der Carbonsäuren IIa bzw. der Ester IIb entstehen in manchen Fällen in untergeordnetem Maße Nebenprodukte, und zwar in Folge einer Überreduktion insbesondere die entsprechenden primären Alkohole, welche gewünschtenfalls in an sich bekannter Weise zu den Aldehyden I oxidiert werden können.

Da die Oxidation dieser Alkohole auch mit den für das erfindungsgemäße Verfahren beschriebenen Katalysatoren gelingt, besteht die Möglichkeit, die Alkohole in das erfindungsgemäße Verfahren rückzuführen, vorteilhafterweise nach Vermischen mit Ausgangsverbindung IIa oder IIb.

In manchen Fällen reagieren die primären Alkohole mit den Carbonsäuren IIa zu Estern oder mit den Estern IIb zu Umesterungsprodukten, welche anhand des erfindungsgemäßen Verfahrens zu Aldehyden I umgesetzt werden können, indem man sie, zweckmäßigerweise nach Vermischen mit frischer Carbonsäure IIa oder Ester IIb, in das Verfahren zurückführt.

Bei der Herstellung von 4-Formyltetrahydropyran beispielsweise können die Nebenprodukte {4-Hydroxymethyltetrahydropyran und Tetrahydropyran-4-carbonsäure(tetrahydropyran-4-yl-methyl) ester) zusammen mit nicht umgesetzter Ausgangsverbindung {Tetrahydropyran-4-carbonsäureester (IIb)} ohne vorherige Auftrennung in das erfindungsgemäße Verfahren rückgeführt werden, da die genannten Komponenten einen höheren Siedepunkt als das gewünschte Verfahrensprodukt 4-Formyltetrahydropyran haben und letzteres demnach leicht abtrennbar ist.

Mit dem erfindungsgemäßen Verfahren lassen sich in der Regel Ausbeuten zwischen 20 und 80% an den Aldehyden I, bei Selektivitäten von 50 bis 90 %, erzielen.

Die Verfahrensprodukte eignen sich als Zwischenprodukte für Herbizide vom Cyclohexenontyp (vgl. z.B. die EP-A 142 741).

### Beispiele

In den folgenden Beispielen wurden teils käufliche, teils eigens hergestellte Katalysatoren eingesetzt.

### A) Herstellung der Katalysatoren

Monoklines Zirkondioxid (Fa. Norton, Akron, Ohio) in Form von Strängen (3 mm Durchmesser) bzw. Manganoxid (MnO, Herstellung aus Mangan(II)-carbonat, vgl. Hollemann-Wiberg, Lehrbuch der anorganischen Chemie, 81.-90. Auflage, Verlag Walter de Gruyter, Berlin, Seite 905) in Form von Tabletten wurde mit einer wäßrigen Lösung des Lanthanid-Element-Nitrats, Yttrium(III)nitrat bzw. von Chrom-(III)-nitrat unter guter Durchmischung getränkt und 2 Stunden bei Raumtemperatur gehalten. Anschließend wurde der so erhaltene Katalysator 15 Stunden bei 120°C getrocknet und anschließend 2 bis 4 Stunden im Luftstrom auf 400 bis 500°C erhitzt.

### B) Herstellung von 4-Formyltetrahydropyran

Pro Stunde wurden 10 Gramm Tetrahydropyran-4-carbonsäuremethylester bei 250 bis 300°C verdampft und zusammen mit 100 Liter Wasserstoff in einem Rohrreaktor von 25 mm Durchmesser an 100 g Katalysator KAT der angegebenen Dotierung und Oberfläche S bei einer Temperatur von T°C umgesetzt. Das kontinuierlich entnommene Rohprodukt wurde kondensiert und quantitativ-gaschromatographisch auf 4-Formyltetrahydropyran hin analysiert.

Die Einzelheiten der durchgeführten Versuche sowie deren Ergebnisse sind in der folgenden Tabelle zusammengestellt.

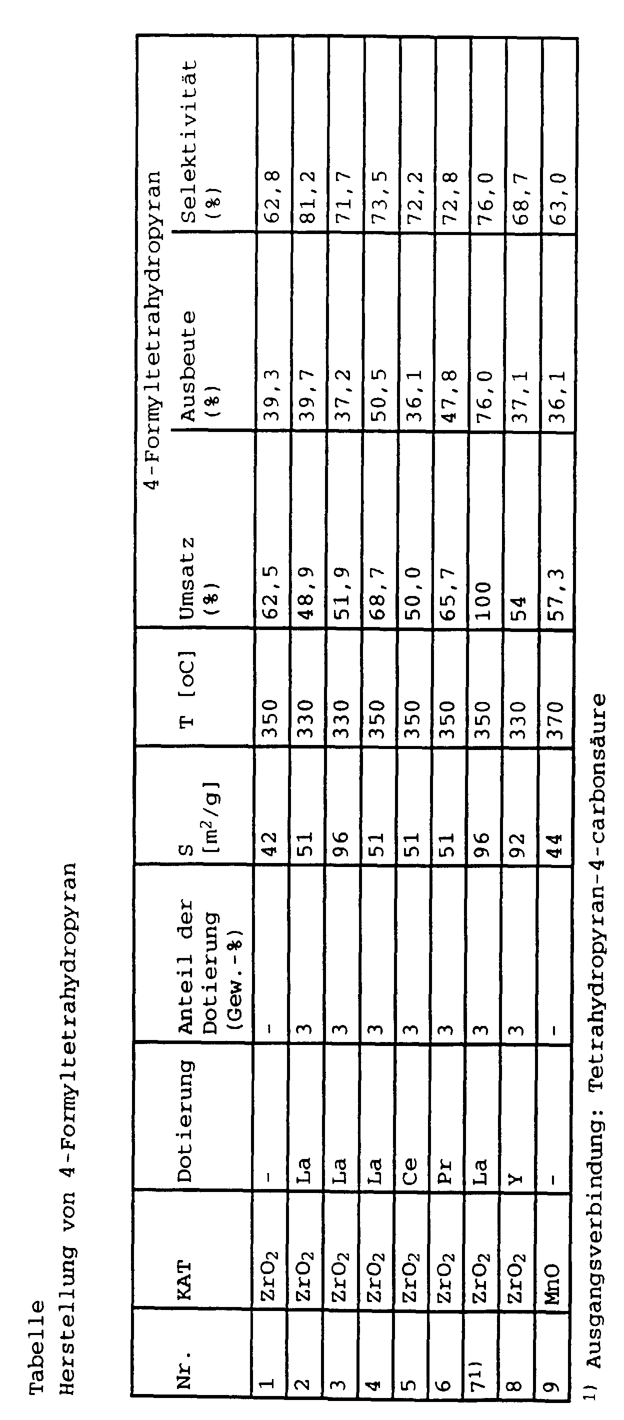

### C) Herstellung von 4-Formyltetrahydropyran im Technikumsmaßstab

Pro Stunde wurden 130 g Tetrahydropyran-4-carbonsäuremethylester bei 250°C verdampft und zusammen mit 1200 1 Wasserstoff in einem Rohrreaktor von 24 mm Durchmesser und 4 m Länge an 1700 ml Katalysator 2 bei einer Temperatur von 330 bis 355°C umgesetzt. Das kontinuierlich entnommene Rohprodukt wurde kondensiert und quantitativ-gaschromatographisch analysiert. Die Versuchsdauer betrug 2700 h. Die mittlere Aldehyd-Ausbeute betrug 52 % (Selektivität: 83 %).

### D) Herstellung von 4-Formyltetrahydropyran (FTHP) im Technikumsmaßstab mit Rückführung der Nebenprodukte

Es wurde wie unter C) verfahren, wobei man aber das kontinuerlich entnommene Rohprodukt nach der Kondensation über einen Zeitraum von 7 Tagen sammelte und anschließend diskontinuierlich destillierte (5 m Sulzer CY-Packung, ca. 50 theoretische Trennstufen). Nach der destillativen Aufarbeitung wurden nicht umgesetzter Tetrahydropyran-4-carbonsäuremethylester (THPE), 4-Hydroxymethyltetrahydropyran (HMTHP) und Tetrahydropyran-4-carbonsäure-(tetrahydropyranylmethyl)-ester (THPPE) in die Hydrierung zurückgeführt. Nach Durchlaufen einiger Rückführzyklen stellten sich folgende stationäre Mengenverhältnisse ein:

Zulaufgemisch (21,8 kg für 7 Tage):
- THPE (frisch): 11,3 kg (78 mol)
- THPE (rückgeführt): 1,0 kg ( 7 mol)
- HMTHP (rückgeführt): 6,7 kg (58 mol)
- THPPE (rückgeführt): 2,1 kg ( 9 mol)
- Sonstige Nebenprod.: 0,7 kg

Destillative Aufarbeitung:
- Fraktion 1: Methanol + Leichtsieder (Sdp. 38 bis 80°C / 300 mbar) 2,5 kg
- Fraktion 2: FTHP (Sdp. 81°C / 30 mbar) 8,0 kg (70 mol)
- Fraktion 3: THPE (Sdp. 94°C / 30 mbar) 1,0 kg ( 7 mol)
- Fraktion 4: HMTHP (Sdp. 118°C / 30 mbar) 6,8 kg (59 mol)
- Fraktion 5: THPPE (Sdp. 165°C / 5 mbar) 2,1 kg ( 9 mol)
Zwischenfraktionen wurden bei der folgenden Destillation erneut eingesetzt und so verbliebene Wertprodukte zurückgewonnen. Die Ausbeute an 4-Formyltetrahydropyran (bezogen auf Frisch-THPE) betrug nach der Destillation 90 % (Reinheit > 99 %).

## Patentansprüche

1. Verfahren zur Herstellung von heterocyclischen Aldehyden der allgemeinen Formel I 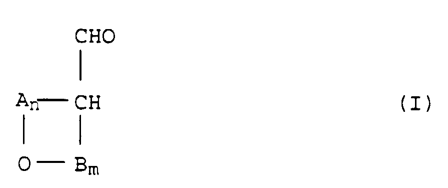 in der die Variablen A und B für Methylengruppen, die ein oder zwei unter den Reaktionsbedingungen inerte Substituenten tragen können, und m und n für 1 bis 5 stehen, wobei die Summe aus m und n größer als 2 ist, dadurch gekennzeichnet, daß man eine Carbonsäure der allgemeinen Formel IIa 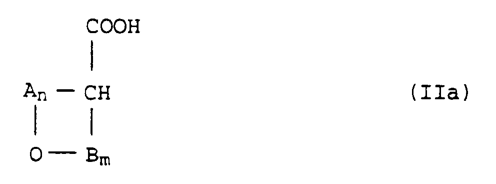 oder einen ihrer Ester IIb, welcher sich von einem C₁- bis C₁₀-Alkohol ableitet, bei 200 bis 450°C an einem Katalysator hydriert.

2. Verfahren nach Anspruch 1, dadurch gekennzeichnet, daß man die Hydrierung in der Gasphase durchführt.

3. Verfahren nach Anspruch 1 oder 2, dadurch gekennzeichnet, daß der Katalysator im wesentlichen aus Zirkoniumdioxid und/oder einem oder mehreren Manganoxiden besteht.

4. Verfahren nach den Ansprüchen 1 bis 3, dadurch gekennzeichnet, daß man einen Katalysator verwendet, welcher 0,1 bis 10 Gew.-% einer oder mehrerer Verbindungen des Chroms und/ oder eines oder mehrerer Elemente der Lanthaniden-Reihe des Periodensystems der Elemente enthält.

5. Verfahren nach Anspruch 4, dadurch gekennzeichnet, daß in dem Katalysator die Verbindungen des Chroms und/oder der Elemente der Lanthaniden-Reihe des Periodensystems der Elemente zumindest teilweise in oxidischer Form vorliegen.

6. Verfahren nach den Ansprüchen 1 bis 5, dadurch gekennzeichnet, daß man einen Katalysator verwendet, welcher im wesentlichen aus Zirkoniumdioxid und 0,1 bis 10 Gew.-% einer Verbindung eines oder mehrerer Elemente der Lanthaniden-Reihe des Periodensystems der Elemente besteht.

7. Verfahren nach den Ansprüchen 1 bis 6, dadurch gekennzeichnet, daß man einen Katalysator verwendet, welcher im wesentlichen aus Zirkoniumdioxid besteht und 0,5 bis 5 Gew.-% Lanthan-(III)-oxid enthält.

8. Verfahren nach den Ansprüchen 1 bis 7, dadurch gekennzeichnet, daß man monoklines Zirkoniumdioxid verwendet.

9. Verfahren zur Herstellung von 4-Formyltetrahydropyran, dadurch gekennzeichnet, daß man Tetrahydropyran-4-carbonsäure oder einen ihrer Ester, welcher sich von einem C₁- bis C₁₀-Alkohol ableitet, in Gegenwart eines Katalysators, welcher im wesentlichen aus Zirkoniumdioxid besteht und 0,1 bis 5 Gew.-% Lanthan-(III)-oxid enthält, hydriert.

10. Verfahren nach Anspruch 1, dadurch gekennzeichnet, daß man anschließend das Wertprodukt (I) aus dem Hydriergemisch abtrennt und die Nebenprodukte
- Alkohol, der dem Aldehyd (I) entspricht, und/oder
- Ester dieses Alkohols mit IIa oder IIb,
gewünschtenfalls zusammen mit Ausgangsverbindung IIa oder IIb, erneut dem Verfahren unterwirft.

## Claims

1. A process for preparing heterocyclic aldehydes of the formula I 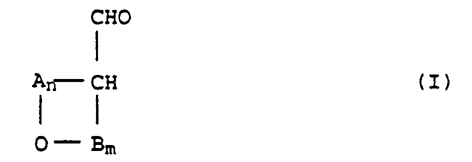 where the variables A and B are each methylene groups which may carry one or two substituents which are inert under the reaction conditions, and m and n are each from 1 to 5, the sum of m and n being greater than 2, which comprises hydrogenating a carboxylic acid of the formula IIa 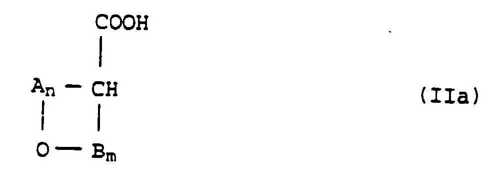 or one of its esters IIb which is derived from a C₁- to C₁₀-alcohol, at from 200 to 450°C over a catalyst.

2. A process as claimed in claim 1, wherein the hydrogenation is carried out in the gas phase.

3. A process as claimed in claim 1 or 2, wherein the catalyst essentially consists of zirconium dioxide and/or one or more manganese oxides.

4. A process as claimed in any of claims 1 to 3, wherein a catalyst is employed which comprises from 0.1 to 10% by weight of one or more compounds of chromium and/or one or more elements of the lanthanide series of the Periodic Table of the Elements.

5. A process as claimed in claim 4, wherein in the catalyst the compounds of chromium and/or the elements of the lanthanide series of the Periodic Table of the Elements are at least in part in oxidized form.

6. A process as claimed in any of claims 1 to 5, wherein a catalyst is used which essentially consists of zirconium dioxide and from 0.1 to 10% by weight of a compound of one or more elements of the lanthanide series of the Periodic Table of the Elements.

7. A process as claimed in any of claims 1 to 6, wherein a catalyst is used which essentially consists of zirconium dioxide and from 0.5 to 5% by weight of lanthanum(III) oxide.

8. A process as claimed in any of claims 1 to 7, wherein monoclinic zirconium dioxide is employed.

9. A process for preparing 4-formyltetrahydropyran, which comprises hydrogenating tetrahydropyran-4-carboxylic acid or one of its esters which is derived from a C₁- to C₁₀-alcohol, in the presence of a catalyst which essentially consists of zirconium dioxide and from 0.1 to 5% by weight of lanthanum(III) oxide.

10. A process as claimed in claim 1, wherein the desired product (I) is subsequently separated off from the hydrogenation mixture, and the by-products
- alcohol which corresponds to the aldehyde (I) and/or
- esters of this alcohol with IIa or IIb,
if desired together with starting compound IIa or IIb, are passed through the process again.

## Revendications

1. Procédé de préparation d'aldéhydes hétérocycliques de formule générale I 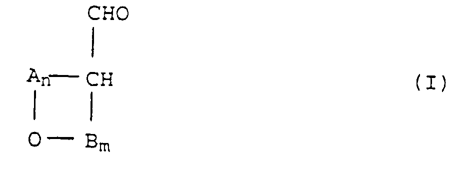 dans laquelle A et B représentent des groupes méthylène qui peuvent porter un ou deux substituants inertes dans les conditions de la réaction, et m et n sont des nombres allant de 1 à 5, la somme de m et n étant supérieure à 2, ce procédé se caractérisant par le fait que l'on hydrogène à des températures de 200 à 450°C sur un catalyseur un acide carboxylique de formule générale IIa 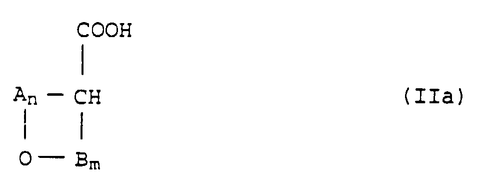 ou l'un de ses esters IIb dérivant d'un alcool en C1-C10.

2. Procédé selon la revendication 1, caractérisé par le fait que l'on procède à l'hydrogénation en phase gazeuse.

3. Procédé selon la revendication 1 ou 2, caractérisé par le fait que le catalyseur consiste essentiellement en dioxyde de zirconium et/ou en un ou plusieurs oxydes du manganèse.

4. Procédé selon la revendication 1 à 3, caractérisé par le fait que l'on utilise un catalyseur contenant 0,1 à 10 % en poids d'un ou plusieurs composés du chrome et/ou d'un ou plusieurs éléments de la série des lanthanides de la Classification Périodique des Eléments.

5. Procédé selon la revendication 4, caractérisé par le fait que, dans le catalyseur, les composés du chrome et/ou des éléments de la série des lanthanides de la Classification Périodique des Eléments sont, en partie au moins, à l'état d'oxydes.

6. Procédé selon les revendications 1 à 5, caractérisé par le fait que l'on utilise un catalyseur qui consiste essentiellement en dioxyde de zirconium et 0,1 à 10 % en poids d'un composé d'un ou plusieurs éléments de la série des lanthanides de la Classification Périodique des Eléments.

7. Procédé selon les revendications 1 à 6, caractérisé par le fait que l'on utilise un catalyseur qui consiste essentiellement en dioxyde de zirconium et contient 0,5 à 5 % en poids d'oxyde de lanthane-III.

8. Procédé selon les revendications 1 à 7, caractérisé par le fait que l'on utilise un dioxyde de zirconium monoclinique.

9. Procédé de préparation du 4-formyltétrahydropyranne, caractérisé par le fait que l'on hydrogène l'acide tétrahydropyranne-4-carboxylique ou l'un de ses esters dérivant d'un alcool en C1-C10, en présence d'un catalyseur consistant essentiellement en dioxyde de zirconium et qui contient 0,1 à 5 % en poids d'oxyde de lanthane-III.

10. Procédé selon la revendication 1, caractérisé par le fait que l'on sépare ensuite le produit recherché I du mélange d'hydrogénation et le cas échéant on renvoie à l'opération les produits d'accompagnement
- l'alcool correspondant à l'aldéhyde (I)
et/ou
- des esters de cet alcool et de IIa ou IIb,
avec le composé de départ IIa ou IIb.
